# EUROPEAN PATENT APPLICATION

(11) **EP 1 836 901 A1**
(43) Date of publication of application: **26.09.2007**
(21) Application number: 06111609.1
(22) Date of filing: 23.03.2006
(51) Int. Cl.: A23K 3/00, A01K 61/00

(54) **Method for producing a feed comprising Artemia nauplii**

(71) Applicant: INVE Technologies NV, 9200 Dendermonde (BE)
(72) Inventor: Grymonpré, Dirk, 9080 Lochristi (BE); Van Nieuwenhove, Luciaan 39/268 Danicha Garden, Amphur Pakkred 11120 Nonthaburi (TH)
(74) Representative: Van Reet, Joseph

(57) **Abstract**

The invention relates to a feed for aquatic organisms that comprises *Artemia* nauplii and to a method for producing such feed. To enable the feed to be stored for a long time, the feed is contained in a hermetically sealed packaging and the nauplii are subjected to a physical sterilisation treatment such as a heat treatment and an irradiation treatment. Prior to the sterilisation treatment, the nauplii are preferably blanched. A large amount of the nauplii were kept physically intact and they had a good buoyancy.

## Description

The present invention relates to a method for producing a feed for aquatic organisms, which feed comprises *Artemia* nauplii contained in a packaging. The aquatic organisms are either fresh or salt water organisms and include in particular fish, shrimp and aquatic pets like ornamental fish, and more particularly larvae thereof.

The availability of suitable live food organisms for farming remains a major problem in the aquaculture or ornamental fish industry. Usually, the most preferred live feeds are brine shrimp *Artemia* nauplii. They provide the best results in terms of fish larvae growth and survival in comparison to artificial diets.

The size range of *Artemia* and its different physical forms (umbrella stage, free-swimming nauplii Instar I, enriched nauplii Instar II and adults) make it very versatile for use in aquaculture.

The availability of live food is considered to be a limiting factor in larviculture of many fish and crustacean species. Larvae of many species have been successfully raised using *Artemia* nauplii. However, there are several major disadvantages that are associated with the use of live food, such as the potential for introduction of a pathogen into the culture system and the amount of labour required for the preparation of the live feed.

It is known that *Artemia* nauplii may in practice be heavily contaminated with bacteria, mainly with *Vibrio* species that are potential fish or shrimp pathogens. *Artemia* nauplii can be considered as a possible carrier of pathogenic bacteria which can cause disease and mortality outbreaks in larval rearing of marine fish or shrimp, especially when the larvae are stressed.

Therefore, many efforts were taken to reduce this bacterial load with different chemotherapeutics (e.g.: WO 96/12407). This bacterial reduction is only effective when the *Artemia* nauplii are immediately fed to the predators after harvesting, which is often the case in aquaculture practices. However, in some aquaculture practices and in most ornamental fish practices, the direct use of *Artemia* nauplii is not feasible and during preparation, storage and transport, the number of pathogenic bacteria in the *Artemia* might again increase.

Most available aquarium feeds, some of which include a number of *Artemia* nauplii, are presented as frozen or gelled feeds. However, freezing and gelling is only useful to preserve the feeds for a limited period of time, but it does not prevent the transfer of pathogenic bacteria to the target species.

Frozen feeds also have the disadvantage that freezing storage is necessary, not only in the case of the trader but also in the case of the aquarium holder. If during transport or storage the feed thaws, it deteriorates rapidly and cannot be used anymore, even if it is frozen again. One cannot tell the freshness or the presence of pathogens or other harmful micro-organisms until it is introduced in the aquarium and harms the aquatic feeders.

Moreover, frozen water inside the nauplii crystallizes and might perforate the cell wall, which increases the risk of leaching. Furthermore, the buoyancy is far from optimal, which is a critical factor especially for larval fish.

Gelled live food organism including *Artemia* nauplii with a synthetic or natural gel former are far from sterile, introduce additional gel material in the aquarium and still need preservatives to store it at room temperatures.

Freeze dried feeds containing *Artemia* nauplii are so far the only acceptable feeds without preservatives that can be stored for long periods and wherein the individual *Artemia* nauplii are still palatable for both fish and shrimp.

However, the high cost of freeze drying often prevents the use of this treatment for commercial operations. Moreover, the buoyancy of freeze dried nauplii is far from optimal. Most nauplii tend to float on the water surface where they are mostly not reachable for small fish larvae. Finally, also the freeze drying treatment does not kill off all pathogens.

An object of the present invention is therefore to provide a new method for manufacturing a feed which contains *Artemia* nauplii, wherein the number of food decay micro-organisms has been reduced so that it can be stored for a longer time at room temperature without requiring the use of a preservative, and wherein also the number of possible pathogens is reduced.

To this end, the *Artemia* nauplii are subjected to a physical sterilisation treatment and the packaging wherein the nauplii are contained is hermetically sealed

The physical sterilisation treatment comprises in particular a heat treatment or an irradiation treatment. Such physical sterilisation treatments are well known and are already applied in the feed industry. However, they were never applied for sterilising *Artemia* nauplii. A common problem in sterilisation processes is that many of these products become mushy or agglomerated or they loose their structural appeal. Due to the severe high temperature, pressure and length of heating, fine textural attributes are difficult to achieve. When small invertebrate organisms are autoclaved, they shrivel up or disintegrate so that they are unrecognizable and unattractive for the predators. Also irradiation treatments can disintegrate small invertebrate organisms.

It has however now been found that, in contrast to for example adult *Artemia,* most of the *Artemia* nauplii keep their original shape and stay physically intact despite the sterilisation process, while other small invertebrate organisms usually disintegrate partly or completely. Adult *Artemia* were found to shrivel up or to disintegrate partly or completely and loose their orange colour so that they are no longer attractive as feed for the predators.

In a preferred embodiment of the method according to the invention the nauplii are first blanched with hot water, having a temperature of at least 73°C, and preferably of at least 80°C, so as to stop enzymatic action in the nauplii before subjecting them to said physical sterilisation treatment.

It has been found that such blanching step has a positive effect on the number of nauplii which stay physically intact after the sterilisation process. Moreover, it has been found that the undesired paler colour of the nauplii, obtained after the blanching step, is converted to the desired red-orange colour during the sterilisation process.

The invention also relates to a feed obtained by the method according to the invention. This feed is characterised in that it comprises physically sterilised *Artemia* nauplii which are contained in a hermetically sealed packaging.

Further advantages and particularities of the present invention will become apparent from the following description of some particular embodiments of the feed and method according to the invention.

For producing the feed according to the invention, *Artemia* nauplii have to be provided. In the present specification and claims, the expression *Artemia* nauplii is intended to embrace both free-swimming nauplii and so-called pre-nauplii, i.e. *Artemia* in the umbrella stage. Such *Artemia* nauplii can be harvested from natural or artifical ponds wherein *Artemia's* appear or are cultured. The harvested organisms comprise mostly all different live stages including nauplii and adult *Artemia.* It is possible to increase the number of nauplii by separating them from the adult *Artemia.* In practice, *Artemia* nauplii are however usually produced starting from *Artemia* cysts. Hatching occurs in about 1-2 days, depending on temperature. For the first few hours, the nauplius stays within a hatching membrane that is attached to the cyst capsule. This is also called the "umbrella stage" in which development of the nauplius stage is completed.

At hatching, the nauplius larva (Instar I) emerges as a free-swimming stage. This stage is about 0.4-0.5 mm in length and brownish-orange in color, due to the presence of yolk material. In a sense, the body of the nauplius larva consists mainly of a head. It has three pairs of "head" appendages -- a pair of small first antennae (antennules), a pair of well-developed second antennae, and a pair of mandibles. There is a large lip-like structure (labrum) covering a ventral mouth. A nauplius eye is present but it is not easily distinguished at this stage.

The posterior end of the nauplius consists of the future trunk. Initially, it is short, undifferentiated, and unsegmented. The nauplius larva does not have a complete digestive tract and does not immediately feed. It relies on stored yolk as an energy source. Depending on temperature, it swims weakly for about 12-20 hrs and then molts into the metanauplius larva (Instar II).

In practice the nauplii Instar I are harvested and fed as such. However, these nauplii usually do not contain the desired nutritional quality for the predators and therefore they can be allowed to develop further to nauplii Instar II, in which the nauplii can actively be fed. This is usually done in an enrichment medium containing desired feed components such as oils, vitamins, etc.

Nauplii Instar I and Instar II are the most suitable nauplii for being fed to the aquatic organisms, while umbrella's can be useful to feed to the smallest predators.

In order to enable to store the nauplii for a prolonged time, the nauplii are packaged in a hermetically sealed packaging and are subjected to a physical sterilisation treatment.

This treatment is intended first of all to reduce the number of pathogenic micro-organisms present on the nauplii, in particular the number of *Vibrio* bacteria. The sterilisation treatment is preferably performed in such a manner that the sterilised feed is substantially free of pathogenic micro-organisms.

The physical sterilisation treatment is further intended to reduce the number of food decay micro-organisms so that the feed can be stored for a longer time in its hermetically sealed packaging. The number of food decay micro-organisms is preferably reduced to such an extent that the physically sterilised and hermetically packaged feed can be stored for at least 6 months, preferably for at least 12 months, and more preferably for at least 18 months at 20 °C, without any substantial food decay. In this way, the nauplii can be kept stored for a sufficiently long time at ambient temperatures, even when the nauplii are kept at temperatures of 30°C or more, which is often the case in places of larval shrimp cultures. Although it is possible to add preservatives, there is no need to add such preservatives to the *Artemia* nauplii as long as they remain in the hermetically sealed packaging. Once, the packaging is opened, they can be stored at temperatures between 0 and 4°C up to 7 days. By adding preservative, it is possible to prolong this cold storage period.

The physical sterilisation treatment is not intended to obtain a completely sterile feed, i.e. a feed which does not contain any viable micro-organisms. In practice, it is only important to achieve a commercially sterile product, i.e. a product that can be stored for a sufficiently long time without decay by micro-organisms. Such a product may for example still contain an amount of heat resistant spores of food decay micro-organisms, but this amount should be sufficiently low to enable the achieve the desired minimum storage period.

A first physical sterilisation treatment which can be used in the method according to the invention is a heat treatment wherein the nauplii are heated to a temperature higher than 100°C. In view of maintaining as much as possible the physical structure or texture of the nauplii, they are preferably heated to a temperature higher than 110°C and more preferably to a temperature higher than 120°C. The heat treatment is preferably conducted at a pressure higher than 1 bar, more preferably at a pressure higher than 1.5 bars and most preferably at a pressure higher than 2 bars. The nauplii are thus preferably autoclaved or retorted. The higher the pressure, the higher the temperature can be applied and the shorter the treatment time. This treatment time is preferably longer than 5 minutes and more preferably longer than 10 minutes.

A second possible sterilisation treatment is an irradiation treatment, in particular an irradiation treatment with gamma irradiation. The degree of sterility depends on the radiation dose received by the nauplii. In a preferred embodiment, this dose comprises at least 5 kGy, preferably at least 15 kGy, more preferably at least 25 kGy and most preferably at least 35 kGy. Prior to the irradiation step, the nauplii are cooled or preferably frozen if they cannot be irradiated immediately. The irradiation step can be performed directly onto the cooled or frozen nauplii.

The nauplii can be subjected to the physical sterilisation treatment before being packaged in the packaging or after being introduced in the packaging but before hermetically sealing the packaging. Packaging of the nauplii or sealing of the packaging has then to be performed under substantially sterile conditions. To avoid having to work under sterile conditions, the nauplii are preferably introduced in their packaging and this packaging is hermetically sealed before the physical sterilisation treatment so that the nauplii are sterilised together with their packaging. The packaging of the nauplii may contain bottles, jars, cans, pouches, bags, etc.

Before subjecting the nauplii to the physical sterilisation treatment, they are preferably blanched with hot water so as to stop the enzymatic action in the nauplii. The hot water has a temperature of at least 73°C and preferably a temperature of at least 80°C. Usually it takes about 1 to about 2 minutes to stop the enzymatic activity. The nauplii are preferably immersed in the hot water.

An important advantage of the blanching step is that, especially when the sterilisation treatment comprises an irradiation step, a larger number of the nauplii remain intact after this sterilisation treatment. A further advantage is that the buoyancy of the nauplii is improved. The blanching step has however also a disadvantage: the colour of the nauplii becomes somewhat paler. This disadvantage can however be obviated by subjecting the blanched nauplii to a heat sterilisation treatment as described hereabove. It has indeed been found that after such a heat treatment, a more orange colour (red-orange) was obtained which is perceived as a benefit by the farmers or aquarium owners.

As appears from the above described method, the feed obtained by this method comprises physically sterilised *Artemia* nauplii which are contained in a hermetically sealed packaging. The nauplii comprised in this feed comprise preferably at least 60%, more preferably at least 75% and most preferably at least 85% physically intact nauplii. The feed preferably comprises the nauplii in an unbound state so that when introduced in the water most of them do not stick together and can be eaten as individual nauplii. The nauplii in the feed may be individually suspended in an aqueous medium. This aqueous medium is in particular formed by the water which adheres to the nauplii after the blanching step or after the rinsing step. Before putting the nauplii in their packaging, they are preferably dewatered by sieving or leaking. Even then, an amount of water adheres to the nauplii so that the feed is still in the form of a pourable or a thick flowable liquid (depending on the amount of water between the nauplii).

In addition to the nauplii, the feed may contain other feed components. However, preferably at least 50% by weight of the dry matter contained in the feed, more preferably 75% by weight of this dry matter is dry matter of the *Artemia* nauplii (either intact or not intact).

The following examples illustrate the sterilisation processes and the effect on the physical appearance of the treated *Artemia* compared to frozen *Artemia.*

### Example 1: sterile Artemia nauplii Instar I (blanching and heat sterilization by autoclave)

6 g *Artemia* cysts GSL-strain are incubated in 3 liter artificial seawater (salinity of 25 ppt) and at a temperature of 28°C. The water is fully aerated and illuminated. After 24 hours the nauplii are hatched (nauplii Instar I) and separated from the shell and other debris.

The nauplii are killed by blanching them during 1 to 2 minutes in water of 85°C. After the heat shock, the nauplii are drained off.

Then, the nauplii are put in a small glass bottle (± 50 ml) and closed with a lid that contains a safety button that pops up as soon as the vacuum disappears in the bottle. The nauplli are not filled till the top, leaving enough space for the air (e.g.: about 20% of the total volume). The packed nauplii are then heated in the autoclave at 121 °C during 15 minutes.

The bottle contains more or less 25 g *Artemia* nauplii (wet weight) corresponding to 1.5 million individuals. The nauplii are intact and obtain red- orange coloration. In the water tanks, buoyancy is better than most artificial diets of the same size that are currently commercially available or frozen nauplii. The nauplii can be preserved in packaging for more 18 months at ambient temperatures. No preservatives or colorants are needed. Once opened, they could be kept in the refrigerator at ± 4°C up to 7 days and still have an acceptable low count of decay organisms if kept under hygienic circumstances.

### Example 2: sterile Artemia nauplii Instar II (blanching and heat sterilization by autoclave)

6 g *Artemia* cysts GSL-strain are incubated in 3 liter artificial seawater (salinity of 25 ppt) and at a temperature of 28°C. The water is fully aerated and illuminated. After 24 hours the nauplii are hatched (nauplii Instar I) and separated from the shell and other debris.

Nauplii Instar I are cultured with an oil emulsion for another 24 hours at a temperature of 28°C in artificial seawater of 25 ppt, after wich they are sieved off and drained.

The same sterilizing procedure is conducted as for nauplii Instar I (see example 1). The bottle contains more or less 25 g *Artemia* nauplii (wet weight) corresponding to 1.5 million individuals. The nauplii are intact and obtain red- orange coloration. In the water tanks, buoyancy is better than most artificial diets of the same size or frozen nauplii. The nauplii can be preserved in packaging for more 18 months at ambient temperatures. No preservatives or colorants are needed. Once opened, they are kept in the refrigerator at ± 4°C up to 7 days and still have an acceptable low count of decay organisms if kept under hygienic circumstances.

### Example 3: sterile Artemia nauplii Instar I (by blanching and irradiation)

6 g *Artemia* cysts GSL-strain are incubated in 3 liter artificial seawater (salinity of 25 ppt) and at a temperature of 28°C. The water is fully aerated and illuminated. After 24 hours the nauplii are hatched (nauplii Instar I) and separated from the shell and other debris.

The nauplii Instar I are blanched during 1 to 2 minutes in water of 85°C. After the heat shock, the nauplii are drained off.

Then, the nauplii are put in a small vessel (± 50 ml) and closed with a lid that contains a safety button that pops up as soon as the vacuum disappears in the bottle. The vessel is vacuumed in a vacuum chamber.

The vessel is frozen until treatment with irradiation is possible.

The vessels with frozen nauplii are then irradiated with gamma-irradiation till 40-45 kGy is aborbed. The nauplii are individual, intact and the orange color is more or less retained. In the water tanks, buoyancy is better than most artificial diet of the same size or frozen nauplii. The nauplii can be preserved in the closed vessel for more than 18 months at ambient temperatures. No preservatives or colorants are needed. Once opened, they could be kept in the refrigerator in the refrigerator at ± 4°C up to 7 days and still have an acceptable low count of decay organisms if kept under hygienic circumstances.

### Example 4: sterile Artemia nauplii Instar II (by blanching and irradiation)

6 g *Artemia* cysts GSL-strain are incubated in 3 liter artificial seawater (salinity of 25 ppt) and at a temperature of 28°C. The water is fully aerated and illuminated. After 24 hours the nauplii are hatched (nauplii Instar I) and separated from the shell and other debris.

Nauplii Instar I are cultured with an oil emulsion for another 24 hours at a temperature of 28°C in artificial seawater of 25 ppt, after wich they are sieved off and drained.

The nauplii Instar II are blanched during 1 to 2 minutes in water of 85°C. After the heat shock, the nauplii are drained off.

Then, the nauplii are put in a small vessel (± 50 ml) and closed with a lid that contains a safety button that pops up as soon as the vacuum disappears in the bottle. The vessel is vacuumed in a vacuum chamber.

The vessel is cold stored until irradiation is possible. The vessels with nauplii are then irradiated with gamma-irradiation till 20-25 kGy is aborbed. The nauplii are individual, intact and the orange color is well retained. In the water tanks, buoyancy is better than any artificial diet of the same size or frozen nauplii. The nauplii can be preserved in the closed vessel for more than 18 months at ambient temperatures. No preservatives or colorants are needed. Once opened, they could be kept in the refrigerator in the refrigerator at ± 4°C up to 7 days and still have an acceptable low count of decay organisms if kept under hygienic circumstances.

**Table 1: Appearance of sterile (autoclaved) Artemia : different stages. Same sterilizing procedures were done with Umbrella's and adult Artemia and the physical characteristics were compared with the nauplii Instar I and Instar II**

| | Complete animals | Pigmentation | Buoyancy |
|---|---|---|---|
| Umbrella's | < 60% | Red-orange | ± buoyant |
| Nauplii Instar I | > 90% | Red-orange | good buoyant |
| Nauplii Instar II | > 90% | Red-orange | good buoyant |
| Adult *Artemia* | < 50% | Pale white | sinking |

**Table 2: Appearance of sterile (irradiated: 25 kGy) Artemia: different stages. Same sterilizing procedures were done with Umbrella's and adult Artemia and the physical characteristics were compared with the nauplii Instar I and Instar II**

| | Complete animals | Pigmentation | Buoyancy |
|---|---|---|---|
| Umbrella's | > 90% | Red-orange | ± buoyant |
| Nauplii Instar I | > 90% | Red-orange | good buoyant |
| Nauplii Instar II | > 90% | Red-orange | good buoyant |
| Adult *Artemia* | < 80% | Pale-white | ± sinking |

**Table 3: appearance of frozen (not sterile) Artemia: different stages**

| | Complete animals | Pigmentation | Buoyancy |
|---|---|---|---|
| Umbrella's | > 90% | Pale orange | sinking |
| Nauplii Instar I | > 90% | Pale orange | sinking |
| Nauplii Instar II | > 90% | Pale orange | sinking |
| Adult *Artemia* | < 90% | brown | sinking |

### Feed experiment

Autoclaved *Artemia* nauplii and irradiated *Artemia* nauplii were fed in a laboratory set-up with shrimp larvae and compared to live *Artemia* nauplii. In this test, 100 white shrimp L. vannamei PL3-stage postlarvae were stocked in 2 L Duran bottles with 1 L treated seawater. Each treatment had 4 replicates. The water temperature was controlled at 29°C by placing the bottles in a Bain-Marie system. Aeration was provided with a Pasteur pipette. Water was exchanged 40% to 60% daily. Food was administered 6 times a day.

For the treatment with live nauplii, *Artemia* hatching and harvesting was done twice a day and the live nauplii were kept in cold-storage for maximum 12 hours. At stage PL9, surviving shrimp were counted.

**Table 4: Survival percentages of shrimp larvae fed Artemia nauplii (test 1)**

| Treatment *Artemia* nauplii | Survival | Stdev |
|---|---|---|
| Live nauplii Instar I | 83 | 7 |
| Dead nauplii: autoclaved: 15 min at 121 °C | 68 | 16 |
| Dead nauplii: irradiated: 18 kGY | 80 | 8 |
| Starvation control | 19 | 1 |

Both dead nauplii autoclaved or irradiated were ingested by the shrimp larvae. The survival of the shrimp larvae tended to be nearly as good as the survival of shrimp larvae fed on the live nauplii and was significantly higher than in the starvation control. This trial proves that it is possible to replace partly or even completely the live nauplii by sterilized nauplii.

**Table 5: Survival percentages of shrimp larvae fed Artemia nauplii (test 2)**

| Treatment *Artemia* nauplii | Survival | Stdev |
|---|---|---|
| Live nauplii Instar I | 86 | 2 |
| Dead nauplii: irradiated: 25 kGy | 79 | 4 |
| Dead nauplii: irradiated: 45 kGy | 80 | 8 |
| Starvation control | 16 | 3 |

Both irradiated (25 and 45 kGy) Artemia nauplii were ingested by the shrimp larvae. Despite the higher irradiation (45 kGy), the survival of the shrimp fed these nauplii was comparable with the survival of the shrimp fed lower irradiated nauplii (25 kGy). Both survival percentages tended to be nearly as good as the survival percentage of the shrimp larvae fed on live nauplii and were significantly higher than the survival percentage of the shrimp in the starvation control. This trial proves that irradiated nauplii could replace partly or completely the live nauplii in feeding shrimp larvae.

## Claims

1. A method for producing a feed for aquatic organisms, which feed comprises *Artemia* nauplii contained in a packaging, **characterised in that** the nauplii are subjected to a physical sterilisation treatment and the packaging wherein the nauplii are contained is hermetically sealed.

2. A method according to claim 1, **characterised in that**, before subjecting the nauplii to said physical sterilisation treatment, the nauplii are packaged and the packaging is hermetically sealed, the nauplii being subjected together with the packaging to the physical sterilisation treatment.

3. A method according to claim 1 or 2, **characterised in that** said physical sterilisation treatment comprises a heat treatment step wherein the nauplii are heated to a temperature higher than 100°C, preferably to a temperature higher than 110°C and more preferably to a temperature higher than 120°C.

4. A method according to claim 3, **characterised in that** said heat treatment is conducted at a pressure higher than 1 bar, preferably at a pressure higher than 1.5 bars and more preferably at a pressure higher than 2 bars.

5. A method according to claim 3 or 4, **characterised in that** the nauplii are subjected to said temperature for at least 5 minutes and preferably for at least 10 minutes.

6. A method according to claim 1 or 2, **characterised in that** said physical sterilisation treatment comprises the step of irradiating the nauplii.

7. A method according to claim 6, **characterised in that** the nauplii are irradiated with a dose of at least 5 kGy, preferably with a dose of at least 15 kGy, more preferably with a dose of at least 25 kGy and most preferably with a dose of at least 35 kGy.

8. A method according to any one of the claims 1 to 7, **characterised in that** before subjecting the nauplii to said physical sterilisation treatment they are blanched with hot water having a temperature of at least 73 °C, and preferably of at least 80 °C, so as to stop enzymatic action in the nauplii.

9. A method according to claim 8, **characterised in that** before being packaged, the nauplii are immersed in said hot water.

10. A method according to any one of the claims 1 to 9, **characterised in that** said nauplii comprise pre-nauplii, nauplii Instar I and/or nauplii Instar II, the nauplii preferably comprising nauplii Instar I and/or nauplii Instar II.

11. A feed obtained by a method according to any one of the claims 1 to 10, **characterised in that** it comprises physically sterilised *Artemia* nauplii contained in a hermetically sealed packaging.

12. A feed according to claim 11, **characterised in that** it comprises said nauplii in an unbound state.

13. A feed according to claim 11 or 12, **characterised in that** it comprises such a reduced amount of food decay micro-organisms that it can be stored for at least 6 months, preferably for at least 12 months and more preferably for at least 18 months at 20°C.

14. A feed according to any one of the claims 11 to 13, **characterised in that** the nauplii comprise at least 60%, preferably at least 75% and more preferably at least 85% of physically intact nauplii.
